# EUROPEAN PATENT SPECIFICATION

(11) **EP 2 011 539 B1**
(45) Date of publication and mention of the grant of the patent: **21.04.2010**
(21) Application number: 08157688.6
(22) Date of filing: 05.06.2008
(51) Int. Cl.: A61M 37/00

(54) **Tattoo needle**
Tatoo-Nadel
Aiguille pour tatouage

(30) Priority: 02.07.2007 CN 200710123547
(43) Date of publication of application: 07.01.2009
(73) Proprietor: Häusler, Wolfgang, District 1, Changping District Beijing 102209 (CN)
(72) Inventor: Häusler, Wolfgang, District 1, Changping District Beijing 102209 (CN)
(74) Representative: Hengelhaupt, Jürgen

(56) References cited:
- CN-Y- 2 765 579
- US-A- 1 630 303
- US-A- 1 699 012
- US-A- 6 030 404
- US-A1- 2004 116 953

## Description

### Field of Invention

The present invention relates to needle for tattooing skin or eyebrow.

### Background of the invention

Skin tattoo is well known, for some people, tattoo is a sign of personalization and beauty. A conventional tattoo needle for tattooing the skin is disclosed in Chinese Utility Model patent number CN2765579Y, as shown in Fig 1, the needle comprises a bar, and a plurality of needle soldered to the bar, each needle have a tapered portion for penetrating the skin. The needles are configured so that the needle tips are in a line, two lines, or a circle defined area, of same height.

US 2004/0116953 A1 discloses needle devices for permanent make-up wherein the needles are provided either in planar fashion or in a V-type profile. Arrangement of the needles in essentially planar arrays is important for pigmenting sharply defined lines.

A tattoo pattern may comprises thin lines, thick lines and color areas, Needles recited in said patent are generally used for tattooing thin line, if thick lines or color areas are to be achieved, the needle may have to be handled to move back and force on the skin, and in this case, it will take a long time to get the tattoo finished.

Moreover, it is very difficult, time consuming, need long years of experience and big portion talent for adding special effects, such as shading to a tattoo pattern, since the transition part of the shading can not be finished which is due to the structure limitation of the current tattoo needle.

It is therefore a need in the prior art to provide a new tattoo needle to settle these problems.

### Summary of the invention

It is an object of the present invention to provide a tattoo needle to improve the efficiency on drawing of lines or filling the color area of a tattoo pattern, and to add new effects, such as shading to a tattoo pattern.

This tattoo needle according to the present invention comprises a bar and a needle assembly, the needle assembly consist of a base portion and a needle portion, the base portion is soldered to the bar, the needle portion consist of a plurality of needles, the needles are so arranged that the needle tips of which fall on the surface of a convex, wherein said convex is part of a sphere.

By utilizing the tattoo needle of the present invention along with a tattoo machine, one movement of tattooing, in stead of repeated movements, would achieve a full dark line, and therefore the total tattooing time is shortened and weak skins are protected in terms of filling and line drawing. Moreover, by using the tattoo needle of the present invention, shading effect can be achieved which is difficult for a common needle of the prior art.

The above and other objects, features and advantages of the present invention will become apparent from the following detailed description taken with the accompanying drawings.

### Brief Description Of The Drawings

Fig 1 is the side view of a conventional tattoo needle.
Fig 2 is the perspective view of a tattoo needle having a circular cross sectional view according to one embodiment of the invention.
Fig 3 is the enlarged top view of the tattoo needle of figure 2.
Fig 4 is the A-Across-sectional view of the tattoo needle of Fig 3.
Fig 5 is the perspective view of a tattoo needle having a triangular cross section needle assembly according to another embodiment of the invention.
Fig 6 is the enlarged top view of the tattoo needle of figure 5.
Fig 7 is the A-A cross-sectional view of the tattoo needle of Fig 6
Fig 8 is the top view of a tattoo needle having an oval cross section needle assembly according to another embodiment of the invention.
Fig 9 is the top view of a tattoo needle having a needle arrangement of a quadrangle cross section needle assembly according to another embodiment of the invention.
Fig 10 is the top view of a tattoo needle having a pentagon cross section needle assembly according to another embodiment of the invention.
Fig 11 is the top view of a tattoo needle having a hexagon cross section needle assembly according to another embodiment of the invention.
Fig 12 is the top view of a tattoo needle having a half round cross section needle assembly according to another embodiment of the invention.
Fig 13 is the top view of a tattoo needle having a rectangle cross section needle assembly according to another embodiment of the invention.
Fig 14 is the top view of a tattoo needle having a rectangle with two corners rounded cross section needle assembly according to another embodiment of the invention.
Fig 15 is the top view of a tattoo needle having a rectangle with all corners rounded cross section needle assembly according to another embodiment of the invention.
Fig 16 is the top view of a tattoo needle having a trapezium cross section needle assembly according to another embodiment of the invention.
Fig 17 is the top view of a tattoo needle having a trapezium with two corners rounded cross section needle assembly according to another embodiment of the invention.
Fig 18 is the top view of a tattoo needle having a trapezium with all corners rounded cross section needle assembly according to another embodiment of the invention.
Fig 19 is the top view of a tattoo needle having a round cornered three-pointed star cross section needle assembly according to another embodiment of the invention.
Fig 20 is the top view of a tattoo needle having a round cornered four-pointed star cross section needle assembly according to another embodiment of the invention.
Fig 21 is the top view of a tattoo needle having a round cornered five-pointed star cross section needle assembly according to another embodiment of the invention.
Fig 22 is the top view of a tattoo needle having a round cornered six-pointed star cross section needle assembly according to another embodiment of the invention.
Fig 23 is the top view of a tattoo needle having a round cornered seven-pointed star cross section needle assembly according to another embodiment of the invention.
Fig 24 is the top view of a tattoo needle having a and round cornered eight-pointed star cross section needle assembly according to another embodiment of the invention.
Fig 25 is the top view of a tattoo needle having a round cornered three-pointed star cross section needle assembly according to another embodiment of the invention.
Fig 26 is the top view of a tattoo needle having a round cornered four-pointed star cross section needle assembly according to another embodiment of the invention.
Fig 27 is the top view of a tattoo needle having a round cornered five-pointed star cross section needle assembly according to another embodiment of the invention.
Fig 28 is the top view of a tattoo needle having a round cornered six-pointed star cross section needle assembly according to another embodiment of the invention.
Fig 29 is the top view of a tattoo needle having a round cornered seven-pointed star cross section needle assembly according to another embodiment of the invention.
Fig 30 is the top view of a tattoo needle having a round cornered eight-pointed star cross section needle assembly according to another embodiment of the invention.

### Detailed Description of the Preferred Embodiments

Referring now to the drawings, the present invention will be understood to relate to an improvement in tattoo needle design.

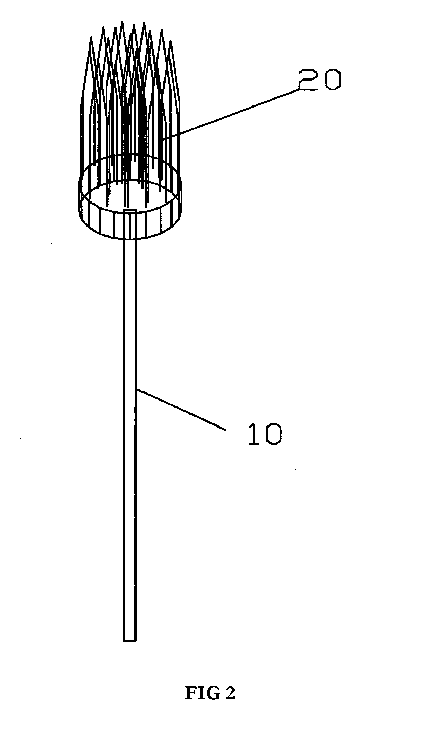

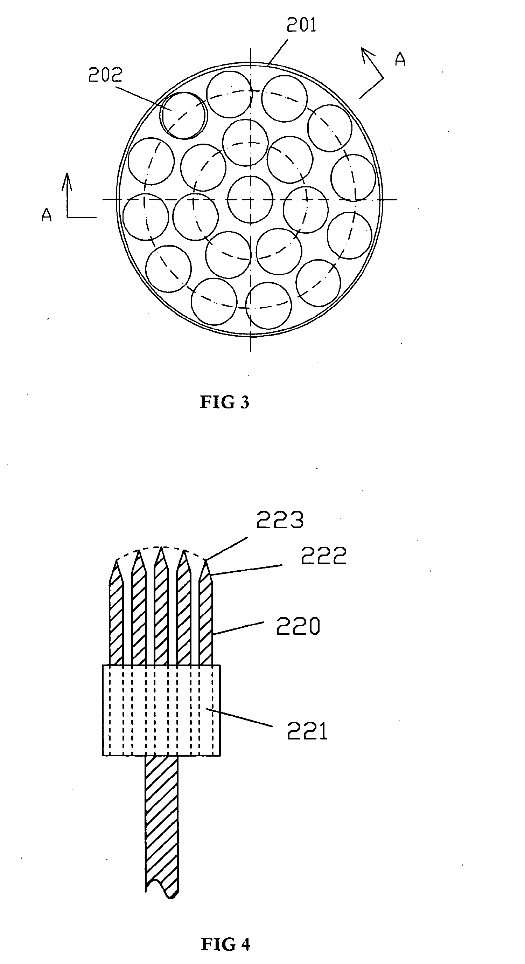

FIG. 2 of the drawings discloses a tattoo needle according to one embodiment of this invention FIG 3 is the top view thereof and FIG 4 is a cross-sectional view thereof. According to FIG2, the tattoo needle comprises a bar 10 and a needle assembly 20, the needle assembly 20 consists of a base portion 201 and a needle portion 202., in this embodiment, the base portion 201 have a circular traversal cross section. The needle portion 202 comprises a plurality of needles 220. Each needle of the plurality of needles comprises a body 221 and a tapered portion 222, the end of tapered portion 222 forms a needle tip 223. Part of The body 221 are soldered together to form the base portion 201 of the needle assembly 20. The needles 220 are so arranged that the needle tips 223 falls on the surface of a sphere. The arrangement can be that the needles are arranged on circles concentric to the circular base portion 201. And height gradient is formed from the midst needle down to the outmost needles, that is, the midst needle or needles is/are the highest and the outmost needles are the lowest.

Other embodiments of this invention intend to give variation of the design of the needle assembly.

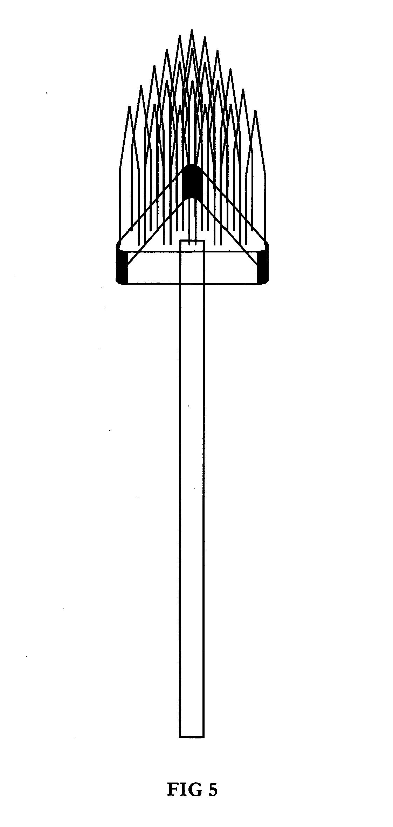

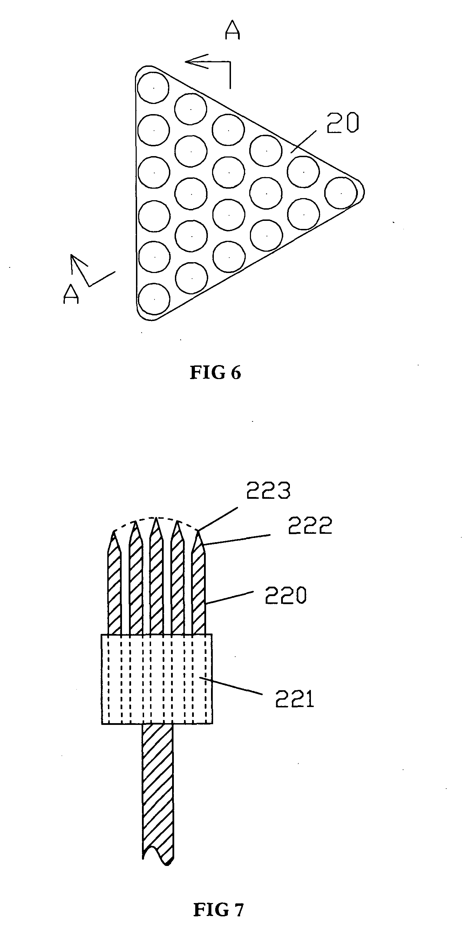

According to figure 5, 6 and 7, the needle assembly 20 may have a rounded triangular cross section. The needles of the needle assembly may be arranged on lines parallel to one side of the rounded triangular cross section. The needle may be so arranged that the needle tips are fall on the surface of a sphere or other shape. And height gradient is formed from the midst needle down to the outmost needles, that is, the midst needle or needles is/are the highest and the outmost needles are the lowest.

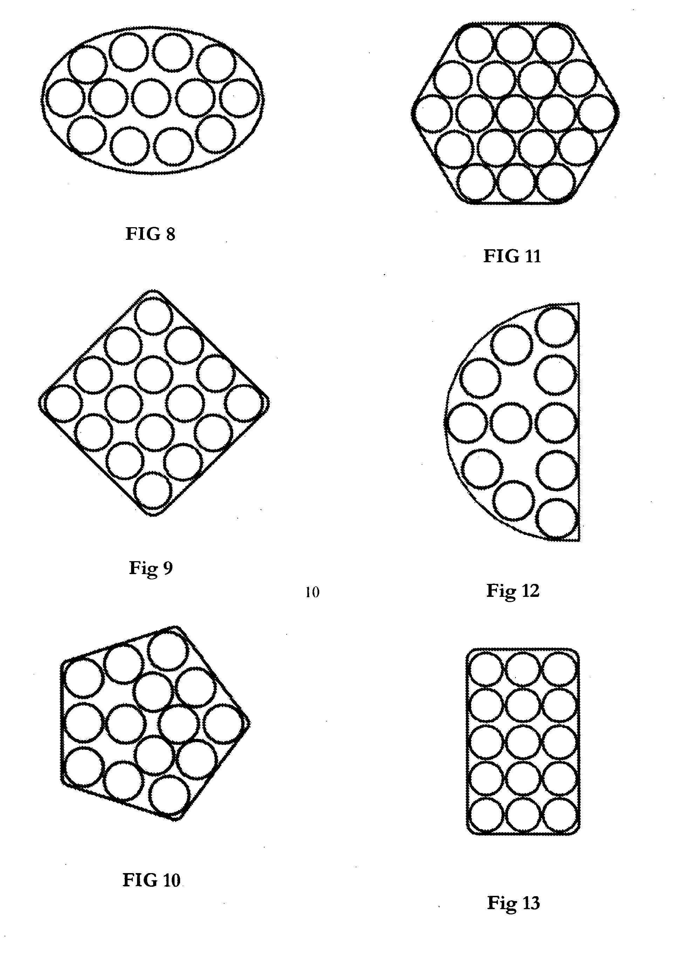

According to figure 8, the needle assembly 20 may have an oval cross section. The needles of the needle assembly may be arranged on ovals concentric to the oval cross section. The needle may be so arranged that the needle tips are fall on the surface of a sphere. And height gradient is formed from the midst needle down to the outmost needles, that is, the midst needle or needles is/are the highest and the outmost needles are the lowest.

Possible shapes of the cross section of the needle assembly are shown in figure 9 to figure 18 which are quadrangle, pentagon, hexagon, half round, rectangle , rectangle with two corners rounded, rectangle with all corners rounded, trapezium, trapezium with two corners rounded, trapezium with all corners rounded respectively. All said needle assemblies possess the characteristic that the needles are so arranged that the needle tips are fall on the surface of a sphere, and height gradient is formed from the midst needle down to the outmost needles, that is, the midst needle or needles is/are the highest and the outmost needles are the lowest.

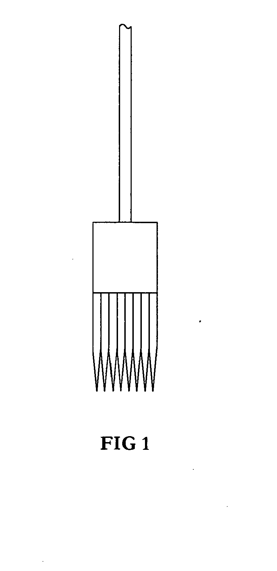

The needles may be arranged according to parallel line like Fig.1, or concentric circles.

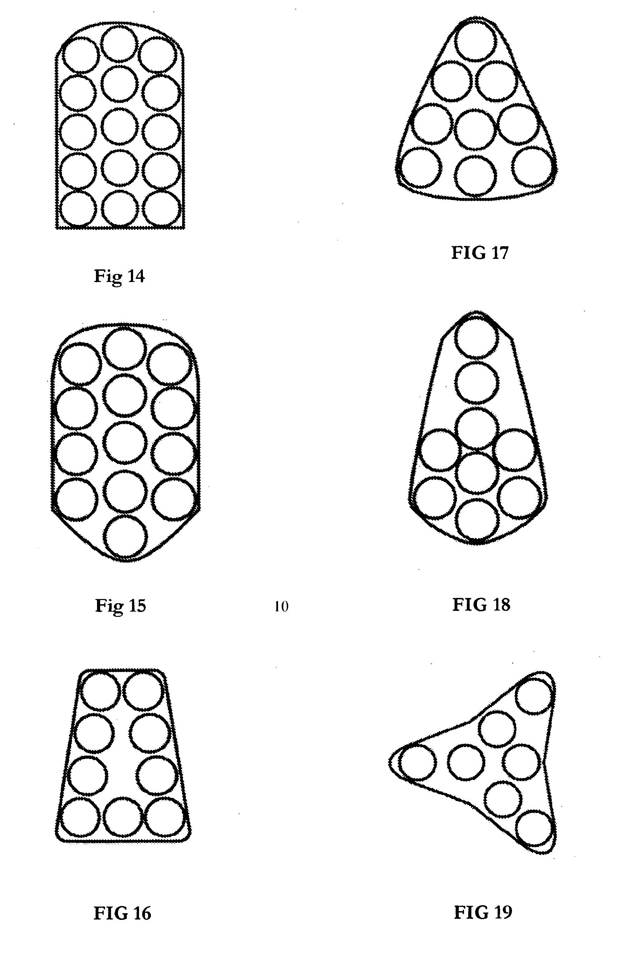

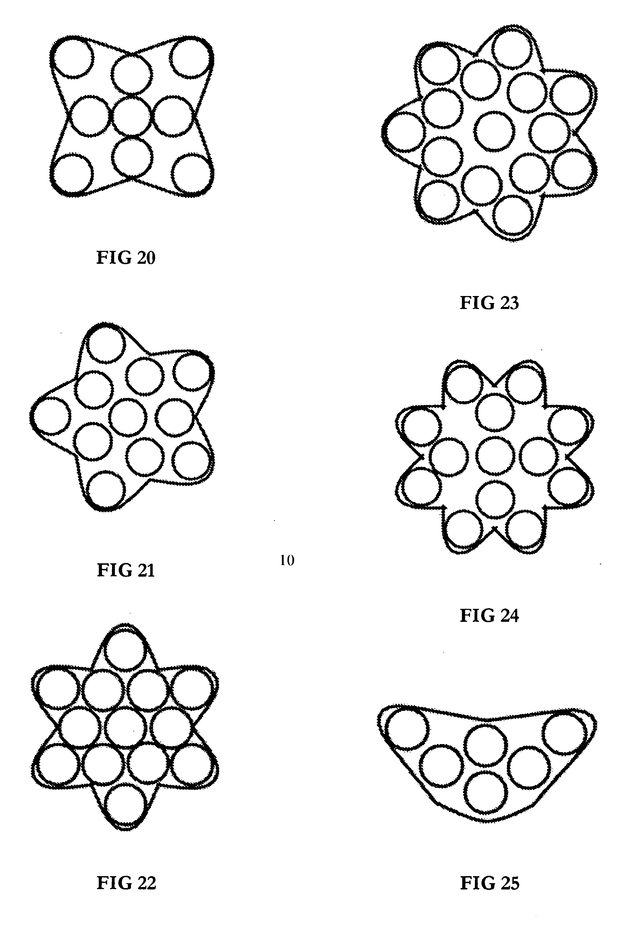

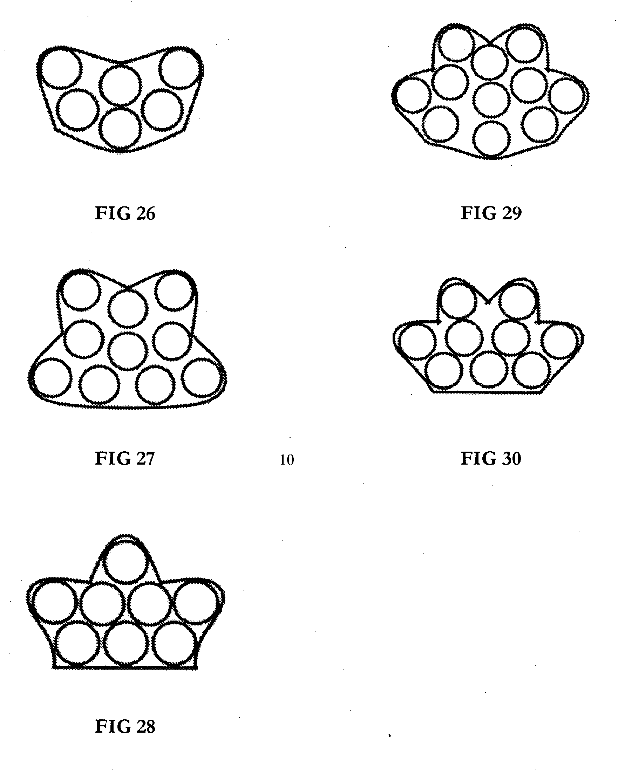

Other possible shape of the cross section of the needle assembly are shown in figure 19 to figure 24 which are round cornered three-pointed star, round cornered four-pointed star, round cornered five-pointed star, round cornered six-pointed star, round cornered seven-pointed star and round cornered eight-pointed star respectively, or part of them or variation of them as shown in figure 25 to 30 respectively.

The needles may be arranged according to parallel line like Fig.01, or concentric circles.

It is can be seen that the needle assembly of the tattoo needle according to this invention can be in various shape, and are independent from size or type, number of needles, needle diameter, needle length or needle taper.

While the invention herein disclosed has been described by means of specific embodiments, numerous modifications and variations could be made thereto by those skilled in the art without departing from the scope of the invention set force in the claims.

## Claims

1. A tattoo needle comprising a bar (10) and a needle assembly (20), wherein the needle assembly (20) consists of a base portion (201) and a needle portion (202) and wherein the base portion (201) is soldered to the bar (10) and the needle portion (202) consists of a plurality of needles, - wherein the needle tips of the needles form the surface of a convex **characterized in that** the convex is part of a sphere.

2. The tattoo needle as recited in claim 1, wherein the curvature of said convex has a height gradient from the midst needle down to the outmost needles.

3. The tattoo needle as recited in claim 1 or 2, wherein the base portion (201) has a circular traversal cross section.

4. The tattoo needle as recited in claim 1 or 2, wherein the base portion (201) has a triangular traversal cross section.

## Patentansprüche

1. Tatoo-Nadel, aufweisend eine Stange (10) und eine Nadelanordnung (20), wobei die Nadelanordnung (20) aus einem Basisteil (201) und einem Nadelteil (202) besteht, und wobei der Basisteil (201) mit der Stange (10) verlötet ist und der Nadelteil (202) aus einer Vielzahl von Nadeln besteht, wobei die Nadelspitzen der Nadeln die Oberfläche einer Konvexität bilden, **dadurch gekennzeichnet, dass** die Konvexität Teil einer Kugel ist.

2. Tatoo-Nadel nach Anspruch 1, wobei die Krümmung der besagten Konvexität einen von der mittleren Nadel hinunter zu den äußersten Nadeln verlaufenden Höhengradienten aufweist.

3. Tatoo-Nadel nach Anspruch 1 oder 2, wobei der Basisteil (201) einen kreisförmigen Querschnitt aufweist.

4. Tatoo-Nadel nach Anspruch 1 oder 2, wobei der Basisteil (201) einen dreieckigen Querschnitt aufweist.

## Revendications

1. Aiguille pour tatouage comprenant une barre (10) à aiguilles et un ensemble (20) aiguille, dans laquelle l'ensemble (20) aiguille consiste en une partie (201) de base et une partie (202) aiguille, et dans laquelle la partie (201) de base est soudée sur la barre (10), et la partie (202) aiguille consiste en une pluralité d'aiguilles, les pointes des aiguilles formant la surface d'un convexe, **caractérisée en ce que** le convexe fait partie d'une sphère.

2. Aiguille pour tatouage selon la revendication 1, dans laquelle la courbure dudit convexe a un gradient de hauteur descendant de l'aiguille centrale jusqu'aux aiguilles extrêmes.

3. Aiguille de tatouage selon la revendication 1 ou 2, dans laquelle la partie (201) de base a une section transversale circulaire.

4. Aiguille de tatouage selon la revendication 1 ou 2, dans laquelle la partie (201) de base a une section transversale triangulaire.
